# EUROPEAN PATENT APPLICATION

(11) **EP 4 145 460 A1**
(43) Date of publication of application: **08.03.2023**
(21) Application number: 21195166.0
(22) Date of filing: 07.09.2021
(51) Int. Cl.: G16H 40/67, G16H 20/10, G16H 20/60, H04L 67/147, H04L 67/00, H04W 76/10, G16H 10/65

(54) **SYSTEM AND METHOD FOR COMMUNICATION BETWEEN A PROGRAMMING INSTANCE AND A PATIENT DEVICE**

(71) Applicant: BIOTRONIK SE & Co. KG, 12359 Berlin (DE)
(72) Inventor: KRUEGER, Daniel, 15741 Bestensee (DE); MUELLER, Jens, 14195 Berlin (DE); DEUTSCHMANN, Hendrik, 12435 Berlin (DE)
(74) Representative: Biotronik Corporate Services SE

(57) **Abstract**

The present disclosure provides a system (1) for communication between a programming instance (2) and a patient device (3), comprising: a programming instance (2) associated with an implant (5) of a patient (P); and a patient device (3) that can be carried along by the patient (P), wherein the programming instance (2) is configured to transmit information directly to the patient device (3) via a first communication interface (30), and/or wherein the programming instance (2) is configured to transmit information to an intermediate instance (4) via a second communication interface (31), the patient device (3) being configured to retrieve the information from the intermediate instance (4) via a third communication interface (32).

## Description

Embodiments of the present disclosure relate to a system for communication between a programming instance and a patient device, a method for communication between a programming instance and a patient device, and a machine-readable medium for executing the method. Embodiments of the present disclosure relate more particularly to a direct interface between an implant programming instance and a patient device.

Medical implants are widely used to replace, support and/or enhance biological structures of patients. Examples of medical implants include, but are not limited to, cardiovascular medical devices such as artificial hearts, artificial heart valves, implantable cardioverter-defibrillators, cardiac pacemakers, and coronary stents. Often, monitoring of the implant and/or the patient's health status is required, as well as a continuous exchange of information between a physician and the patient. This can be time consuming and/or costly.

In light of the above, a system for communication between a programming instance and a patient device, a method for communication between a programming instance and a patient device, and a machine-readable medium for executing the method that overcome at least some of the problems in the art are beneficial.

It is an object of the present disclosure to provide a system for communication between a programming instance and a patient device, a method for communication between a programming instance and a patient device, and a machine-readable medium for executing the method that can improve a communication between a physician and a patient.

The objects are solved by the features of the independent claims. Preferred embodiments are defined in the dependent claims.

According to an independent aspect of the present disclosure, a system for communication between a programming instance and a patient device is provided. The system includes a programming instance associated with an implant of a patient, and a patient device that can be carried along by the patient.

According to some embodiments, which can be combined with other embodiments described herein, the programming instance is configured to transmit information directly to the patient device via a first communication interface.

Additionally, or alternatively, the programming instance is configured to transmit information to an intermediate instance via a second communication interface, the patient device being configured to retrieve the information from the intermediate instance via a third communication interface. The second communication interface may be different from the third communication interface.

According to some embodiments, which can be combined with other embodiments described herein, the programming instance is configured to transmit the information to the patient device during an implantation of the implant and/or a follow-up session after the implantation.

According to some embodiments, which can be combined with other embodiments described herein, the programming instance is configured to transmit the information to the patient device in real-time.

According to some embodiments, which can be combined with other embodiments described herein, the programming instance is configured to transmit the information to the patient device delayed and/or at a predetermined time.

According to some embodiments, which can be combined with other embodiments described herein, the first communication interface and/or the second communication interface and/or the second communication interface is a wireless communication interface. However, the present disclosure is not limited thereto and at least one of the first communication interface, the second communication interface and the third communication interface can be a wired communication interface.

According to some embodiments, which can be combined with other embodiments described herein, the first communication interface and/or the second communication interface and/or the second communication interface is configured for communication via at least one communications network, in particular the Internet.

Preferably, the at least one communications network includes, or is, a local area network and/or a wide area network.

Preferably, the wide area network is configured for at least one of Global System for Mobile Communications (GSM), General Package Radio Service (GPRS), Enhanced Data Rates for GSM Evolution (EDGE), Universal Mobile Telecommunications System (UMTS), Long-Term Evolution (LTE), and Fifth Generation Technology Standard (5G).

According to some embodiments, which can be combined with other embodiments described herein, the programming instance is a medical support device for medical personnel, in particular a physician.

Preferably, the programming instance is a mobile terminal of a physician and/or a web of the physician and/or a web service used by the physician, e.g. a remote programming server with a web interface facing the physician.

According to some embodiments, which can be combined with other embodiments described herein, the patient device is a mobile terminal.

Preferably, the mobile terminal of the patient and/or the mobile terminal of the physician is selected from the group including (or consisting of) a smartphone, a personal digital assistant, a tablet, a notebook, a smart watch, any device with a web browser, and smart glasses.

According to some embodiments, which can be combined with other embodiments described herein, the intermediate instance includes at least one of a network and/or a server and/or a remote network service.

According to some embodiments, which can be combined with other embodiments described herein, the information provided by the programming instance includes at least one of a next follow-up date, medication information, a checklist, nutrition information, a digital version of an implant pass, and an emergency contact. However, the present disclosure is not limited thereto, and the information may include other information for the patient to considered regarding the implant.

According to another independent aspect of the present disclosure, a method for communication between a programming instance and a patient device is provided. The method includes transmitting, by a programming instance associated with an implant of a patient, information directly to a patient device that can be carried along by the patient via a first communication interface.

Additionally, or alternatively, the method includes transmitting, by the programming instance, information to an intermediate instance via a second communication interface, the patient device retrieving the information from the intermediate instance via a third communication interface.

Embodiments are also directed at systems/devices for carrying out the disclosed methods and include system/device aspects for performing each described method aspect. These method aspects may be performed by way of hardware components, a computer programmed by appropriate software, by any combination of the two or in any other manner. Furthermore, embodiments according to the invention are also directed at methods for operating the described device/system. It includes method aspects for carrying out every function of the device/system.

According to another independent aspect of the present disclosure, a machine-readable medium is provided. The machine-readable medium includes instructions executable by one or more processors to implement the method for communication between a programming instance and a patient device of the embodiments of the present disclosure.

The (e.g. non-transitory) machine readable medium may include, for example, optical media such as CD-ROMs and digital video disks (DVDs), and semiconductor memory devices such as Electrically Programmable Read-Only Memory (EPROM), and Electrically Erasable Programmable Read-Only Memory (EEPROM). The machine-readable medium may be used to tangibly retain computer program instructions or code organized into one or more modules and written in any desired computer programming language. When executed by, for example, one or more processors such computer program code may implement one or more of the methods described herein.

So that the manner in which the above recited features of the present disclosure can be understood in detail, a more particular description of the disclosure, briefly summarized above, may be had by reference to embodiments. The accompanying drawings relate to embodiments of the disclosure and are described in the following:
- FIG. 1: shows a schematic view of a system for a direct link communication between a programming instance and a patient device according to embodiments of the present disclosure;
- FIG. 2: shows a schematic view of a system for communication between a programming instance and a patient device using an intermediate instance according to further embodiments of the present disclosure; and
- FIG. 3: shows a schematic view of a system for communication between a programming instance and a patient device using a direct link and/or a intermediate instance according to further embodiments of the present disclosure.

Reference will now be made in detail to the various embodiments of the disclosure, one or more examples of which are illustrated in the figures. Within the following description of the drawings, the same reference numbers refer to same components. Generally, only the differences with respect to individual embodiments are described. Each example is provided by way of explanation of the disclosure and is not meant as a limitation of the disclosure. Further, features illustrated or described as part of one embodiment can be used on or in conjunction with other embodiments to yield yet a further embodiment. It is intended that the description includes such modifications and variations.

Often, monitoring of an implant and/or the patient's health status is required, as well as a continuous exchange of information between a physician and the patient. This can be time consuming and/or costly.

According to the embodiments of the present disclosure, the programming instance is configured to transmit information directly to the patient device e.g. via a wireless communication interface and/or the programming device is configured to transmit information to an intermediate instance, such as a network, wherein the patient device is configured to retrieve the information from the intermediate instance e.g. via a wireless communication interface (e.g., the programming instance and the patient device may be connected to the intermediate instance via an Internet connection).

In this way, the present disclosure provides an interface for direct data exchange between an implant programming instance (programming device) and a patient device. This enables the user, in particular the physician, to enter and exchange information. By means of the patient device, the information can be received and displayed. Furthermore, the intermediate instance, such as a remote network service, can receive data from the programming instance and send it to the patient device.

The programming instance enables physicians to send information, such as a next follow-up appointment, medication, post-implantation checklists, nutritional information, emergency contacts, instructions to be observed by the patient and the like, to the patient digitally, passively and very quickly.

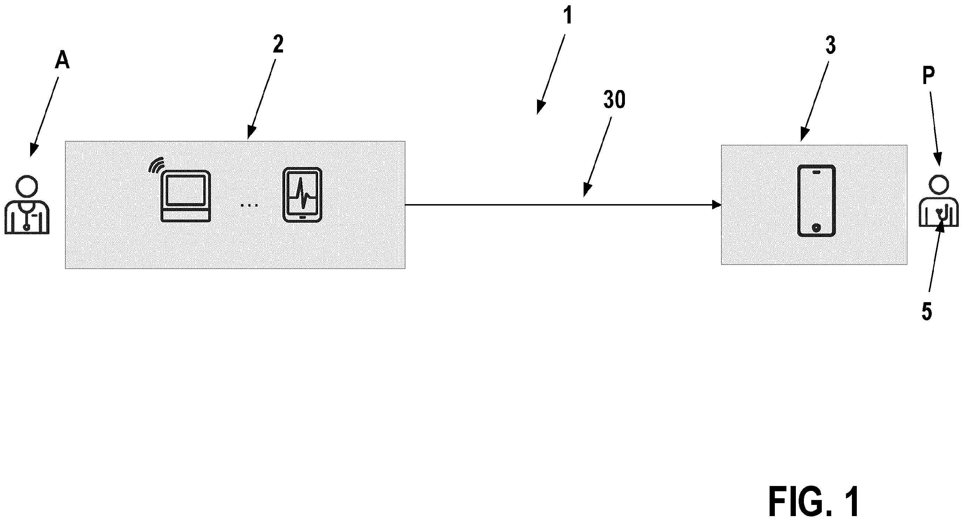

FIG. 1 shows a schematic view of a system 1 for communication between a programming instance 2 and a patient device 3 according to embodiments of the present disclosure.

The programming instance 2 is configured to transmit information directly to the patient device 3 via a first communication interface 30.

The programming instance 2 is associated with an implant 5 of a patient P. The word "associated" means that the programming instance 2 and the implant 5 are related in a sense that the information sent by the programming instance 2 directly and/or indirectly concerns or affects the implant 5. For example, the information sent by the programming instance 2 may include a next follow-up appointment, medication, post-implantation checklists, nutritional information, emergency contacts, instructions to be observed by the patient P, a digital version of an implant pass, and the like.

Examples of medical implants 5 include, but are not limited to, cardiovascular medical devices such as artificial hearts, artificial heart valves, implantable cardioverter-defibrillators, cardiac pacemakers, cardiac monitors and coronary stents.

The programming instance 2 may be a medical support device for medical personnel, such as a physician. The programming instance 2 may have a user interface configured to receive a user input regarding the information to be sent to the patient device 3. In particular, the physician A may enter and/or select the information which are to be sent to the patient device 3 into the user interface of the programming instance 2.

The user interface of the programming instance 2 may include a touch user interface and/or a voice user interface. For example, the user interface may include a touch screen configured to receive a touch input from the physician A. Additionally, or alternatively, the user interface may be configured to receive a voice input from the physician A.

The patient device 3 may be configured to output the information received from the programming instance 2 to the patient P. In some embodiments, the patient device 3 may have a user interface configured to output the received information visually e.g. on a display of the patient device 3 and/or auditory e.g. using a loudspeaker of the patient device 3.

In some embodiments, the patient device 3 may be a mobile terminal of the patient P. Additionally, or alternatively, the programming instance 2 may be a mobile terminal of the physician A. The mobile terminal may be a smartphone, a personal digital assistant, a tablet, a notebook, a smart watch, smart glasses, or a web service. However, the present disclosure is not limited thereto, and other wearables can be used as the patient device 3 and/or the programming instance 2.

In the example of FIG. 1, the programming instance 2 is configured to transmit information directly to the patient device 3 via the first communication interface 30. In some embodiments, the first communication interface 30 can be a wireless communication interface.

The first communication interface 30 may be configured for communication via at least one communications network, such as a local area network (LAN), a Bluetooth connection, an NFC connection and/or a wide area network (WAN), in particular the Internet. The programming instance communicating with the patient device via WAN/Internet is very meaningful if the programming instance is a web service.

Additionally or alternatively, data may be included in a QR-Code which can be scanned by the patient device from the screen of the programming instance.

Thus, according to this example of the present disclosure, it is possible to directly transmit the information in real-time, i.e. without delay, from the programming instance 2 to the patient device 3 e.g. during an implantation or follow-up examination. For this purpose, the programming instance 2 and the patient device 3 are preferably directly connected via standard wireless communication technology.

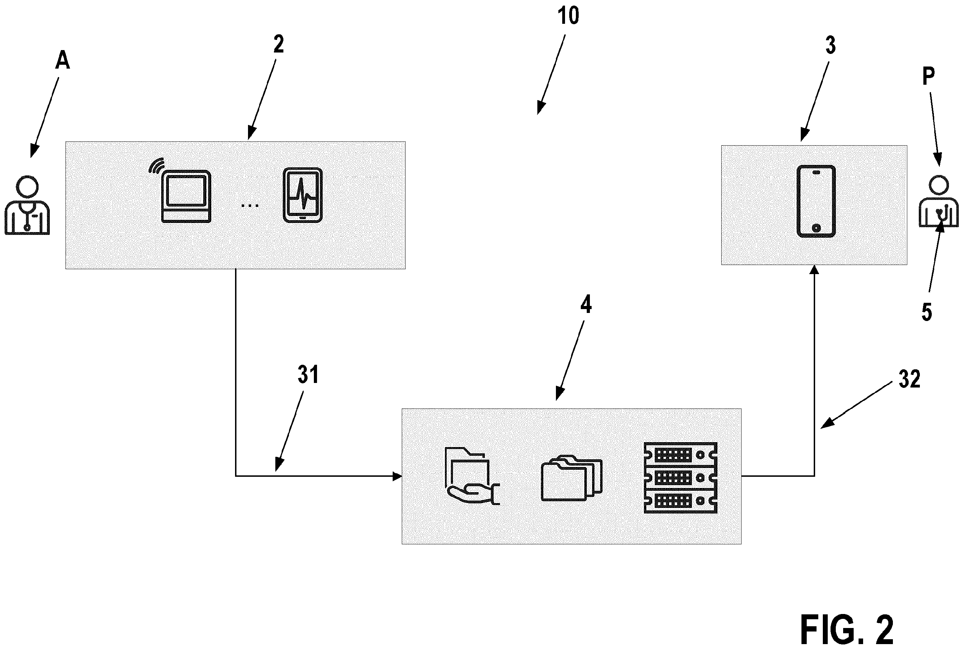

FIG. 2 shows a schematic view of a system 10 for communication between a programming instance 2 and a patient device 3 according to further embodiments of the present disclosure.

In the example of FIG. 2, the programming instance 2 is configured to transmit information to an intermediate instance 4 via a second communication interface 31, the patient device 3 being configured to retrieve the information from the intermediate instance 4 via a third communication interface 32. Accordingly, the programming instance 2 transmits the information indirectly to the patient device 3 via the intermediate instance 4, such as a remote network or remote network service.

In this embodiment, the programming instance 2 and the patient device 3 may be connected to the intermediate instance 4 via an Internet connection.

In some embodiment, the programming instance 2 is configured to transmit the information to the patient device 3 delayed and/or at a predetermined time. For example, the programming instance 2 may transmit the information to the intermediate instance 4 and the intermediate instance 4 may delay the forwarding of the information to the patient device 3 e.g. based on a corresponding delay instruction from the programming instance 2. Accordingly, the patient device 3 does not receive the information in real-time but asynchronously.

In the example of FIG. 2, the programming instance 2 is configured to transmit the information to the intermediate instance 4 via the second communication interface 31. In some embodiments, the second communication interface 31 can be a wireless communication interface. The second communication interface 31 may be configured for communication via at least one communications network, such as a local area network (LAN) and/or a wide area network (WAN), in particular the Internet.

The patient device 3 is configured to receive the information from the intermediate instance 4 via the third communication interface 32. The third communication interface 32 may be configured for communication via at least one communications network, such as a local area network (LAN) and/or a wide area network (WAN), in particular the Internet.

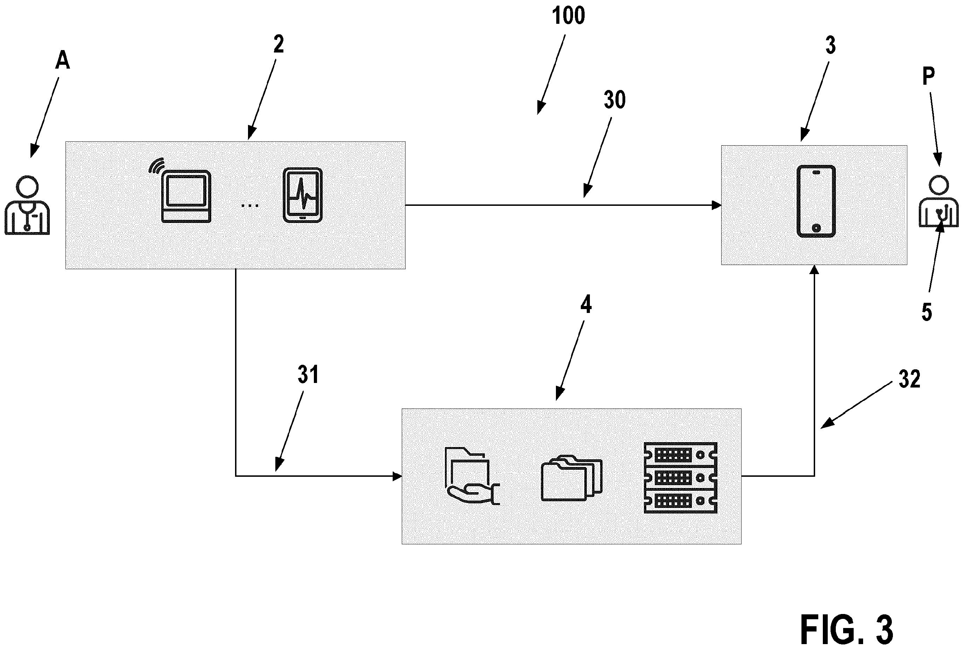

FIG. 3 shows a schematic view of a system 100 for communication between a programming instance 2 and a patient device 3 according to further embodiments of the present disclosure.

The system 100 of FIG. 3 combines the features of the systems of FIGs. 1 and 2. In particular, the programming instance 2 of the system 100 is configured to transmit information directly to the patient device 3 via the first communication interface 30, and is further configured to transmit information to the intermediate instance 4 via the second communication interface 31, the patient device 3 being configured to retrieve the information from the intermediate instance 4 via the third communication interface 32.

The embodiments of the present disclosure can help physicians share important information with the patient and document the handover of the information in one step (no additional paper printout, no easy loss of a paper printout when the information is in digital form). Thus, for remote examinations where the physician and patient are in different locations, there is a convenient way to share important information with the patient digitally, as there is no need for a paper handover. This enables cost savings for clinics and physician practices by minimizing the time for reconfiguration/follow-up of active medical implants 5. Furthermore, an increase in the patient well-being, therapy quality and individual care is achieved.

While the foregoing is directed to embodiments of the disclosure, other and further embodiments of the disclosure may be devised without departing from the basic scope thereof, and the scope thereof is determined by the claims that follow.

## Claims

1. A system (1, 10, 100) for communication between a programming instance (2) and a patient device (3), comprising:
a programming instance (2) associated with an implant (5) of a patient (P); and
a patient device (3) that can be carried along by the patient (P),
wherein the programming instance (2) is configured to transmit information directly to the patient device (3) via a first communication interface (30), and/or
wherein the programming instance (2) is configured to transmit information to an intermediate instance (4) via a second communication interface (31), the patient device (3) being configured to retrieve the information from the intermediate instance (4) via a third communication interface (32).

2. The system (1, 10, 100) of claim 1, wherein the programming instance (2) is configured to transmit the information to the patient device (3) during an implantation of the implant (5) and/or a follow-up session after the implantation.

3. The system (1, 100) of claim 1 or 2, wherein the programming instance (2) is configured to transmit the information to the patient device (3) in real-time.

4. The system (10, 100) of any one of claims 1 to 3, wherein the programming instance (2) is configured to transmit the information to the patient device (3) delayed and/or at a predetermined time.

5. The system (1, 10, 100) of any one of claims 1 to 4, wherein the first communication interface (30) and/or the second communication interface (31) and/or the third communication interface (32) is a wireless communication interface.

6. The system (1, 10, 100) of any one of claims 1 to 5, wherein the first communication interface (30) and/or the second communication interface (31) and/or the third communication interface (32) is configured for communication via at least one communications network, in particular a local area network and/or a wide area network.

7. The system (1, 10, 100) of claim 6, wherein the wide area network is configured for at least one of Global System for Mobile Communications (GSM), General Package Radio Service (GPRS), Enhanced Data Rates for GSM Evolution (EDGE), Universal Mobile Telecommunications System (UMTS), Long-Term Evolution (LTE), and Fifth Generation Technology Standard (5G).

8. The system (1, 10, 100) of any one of claims 1 to 7, wherein the programming instance (2) is a medical support device for medical personnel.

9. The system (1, 10, 100) of any one of claims 1 to 8, wherein the patient device (3) is a mobile terminal.

10. The system (1, 10, 100) of claim 9, wherein the mobile terminal is selected from the group consisting of a smartphone, a personal digital assistant, a tablet, a notebook, a smart watch, and smart glasses.

11. The system (10, 100) of any one of claims 1 to 10, wherein the intermediate instance (4) includes, or is, a network and/or a server.

12. The system (1, 10, 100) of any one of claims 1 to 11, wherein the information provided by the programming instance (2) includes at least one of a next follow-up date, medication information, a checklist, nutrition information, a digital version of an implant pass, and an emergency contact.

13. A method for communication between a programming instance (2) and a patient device (3), comprising:
transmitting, by a programming instance (2) associated with an implant (5) of a patient (P), information directly to a patient device (3) that can be carried along by the patient (P) via a first communication interface (30), and/or
transmitting, by the programming instance (2), information to an intermediate instance (4) via a second communication interface (31), the patient device (3) retrieving the information from the intermediate instance (4) via a third communication interface (32).

14. A machine-readable medium, comprising instructions executable by one or more processors to implement the method for communication between a programming instance (2) and a patient device (3) of claim 13.
